(19) Europäisches Patentamt
European Patent Office
Office européen des brevets

(11) Publication number: 0 465 738 A2

(12) EUROPEAN PATENT APPLICATION

(21) Application number: 90307504.2

(22) Date of filing: 10.07.90

(51) Int. Cl.5: C08G 59/32, C07D 303/24, C07C 39/15, C07C 43/295

(43) Date of publication of application: 15.01.92 Bulletin 92/03

(84) Designated Contracting States: DE FR GB IT

(71) Applicant: DAINIPPON INK AND CHEMICALS, INC.
35-58, Sakashita 3-chome
Itabashi-ku Tokyo(JP)

(72) Inventor: Nakamura, Shuji
4-4 Tatsumidai Higashi
Ichihara-shi, Chiba-ken(JP)
Inventor: Miyazawa, Kenshi
4-4 Tatsumidai Higashi
Ichihara-shi, Chiba-ken(JP)

(74) Representative: Myerscough, Philip Boyd et al
J.A.Kemp & Co. 14, South Square Gray's Inn
London, WC1R 5EU(GB)

(54) **Process for producing tetraepoxy resins, epoxy resin compositions, tetraepoxy resins and tetrahydric phenol compounds.**

(57) An epoxy resin represented by formula

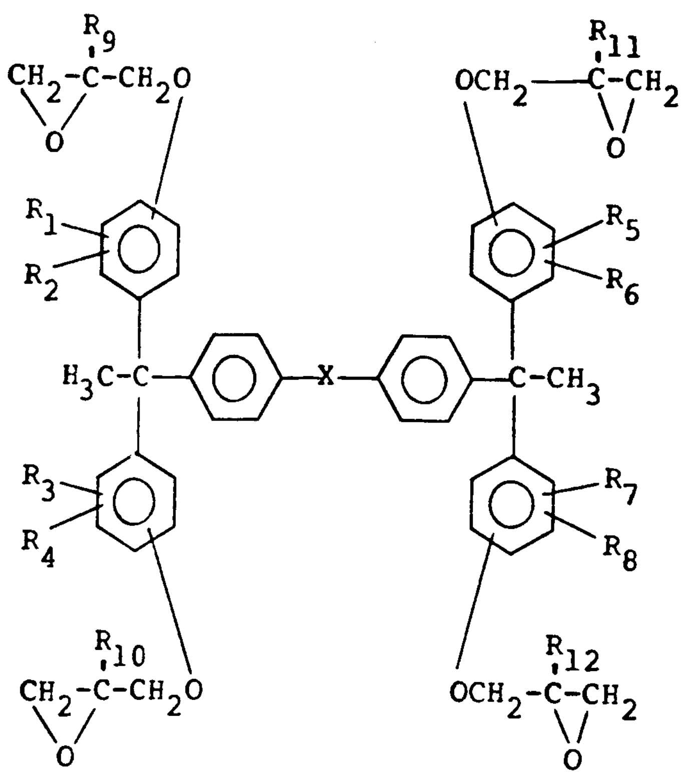

wherein $R_1$ to $R_{12}$ each denote a hydrogen atom or an alkyl group having 1 to 3 carbon atoms, and X

EP 0 465 738 A2

denotes an oxygen atom, a sulfur atom, a methylene group or an ethylene group.

This invention relates to a process for producing an epoxy resin, an epoxy resin composition, a tetraepoxy resin and a tetrahydric phenol compound.

Epoxy resins are generally formed into cured products excellent in mechanical properties, water resistance, chemical resistance, heat resistance and electrical properties, and find wide acceptance in adhesives, paints, laminates, molding materials, and so forth.

Tetraepoxy resins described in European Laid-open Patent No. 304125, Japanese Laid-open Patent Application Nos. 1,718/1983, 90,572/1983 and 150,581/1983, and U.S. Patent No. 4,672,103 are well known.

Since the epoxy resins described in Japanese Laid-open Patent Application Nos. 70,524/1989 and 1,718/1983 have however only four aromatic rings, there are obtained only cured products having insufficient water resistance.

The epoxy resin described in U.S. Patent No. 4,674,103 is a mixture with an oligomer and its cured product is poor in strength and toughness. Even if the tetraepoxy resin can be isolated, the alkylene group to bind the two aromatic rings is long and has a hydroxyl group, so that the cured product is poor in heat resistance and water resistance. Moreover, since the epoxy resins described in Japanese Laid-open Patent Application Nos. 90,572/1983 and 150,581/1983 have only five aromatic rings, the cured product having insufficient water resistance is afforded. Namely, a tetraepoxy resin that gives a cured product excellent in all of heat resistance, toughness and water resistance has not been yet provided. The advent of such tetraepoxy resin has been long demanded.

In the above circumstances, the present inventors have made extensive investigations, and consequently found that a cured product of a tetraepoxy resin with a specific structure having six aromatic rings and four epoxy groups meet the aforesaid requirements. This finding has led to completion of this invention.

This invention thus provides a process for producing a tetraepoxy resin by the reaction of a tetrahydric phenol compound with epichlorohydrin or betamethylepichlorohydrin, characterized in that the tetrahydric phenol compound is a tetrahydric phenol compound represented by formula (I)

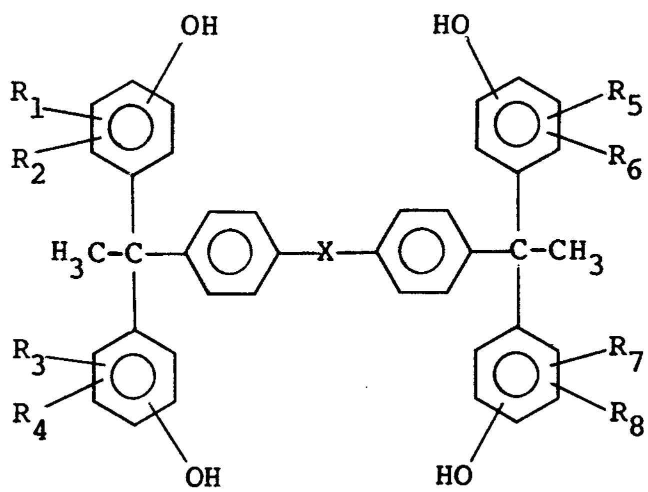

(I)

wherein $R_1$ to $R_8$ each denote a hydrogen atom or an alkyl group having 1 to 3 carbon atoms, and X denotes an oxygen atom, a sulfur atom, a methylene group or an ethylene group, and
an epoxy resin composition comprising a tetraepoxy resin and a curing agent, said epoxy resin being represented by formula (II)

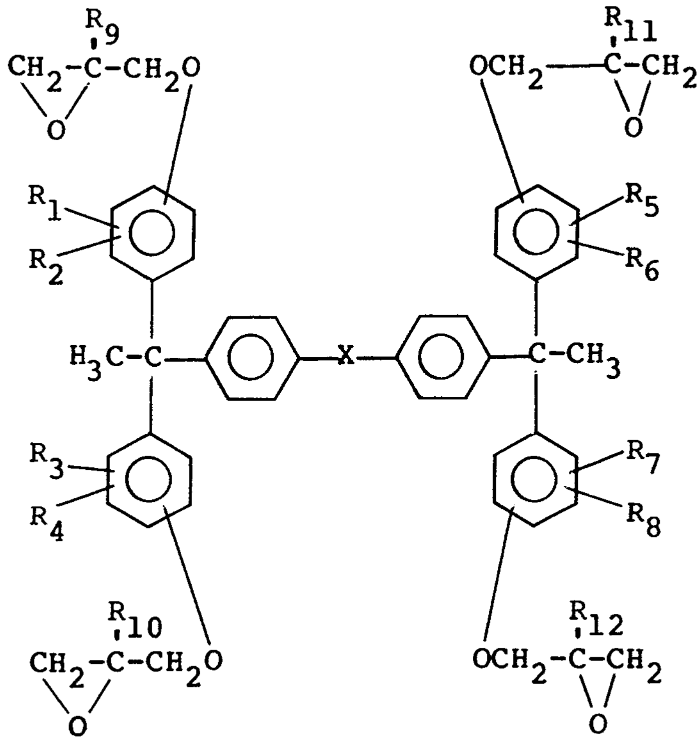

(II)

wherein $R_1$ to $R_{12}$ each denote a hydrogen atom or an alkyl group having 1 to 3 carbon atoms, and X denotes an oxygen atom, a sulfur atom, a methylene group or an ethylene group.

The tetraepoxy resin of this invention can be obtained by the reaction of a tetrahydric phenol compound represented by formula (I)

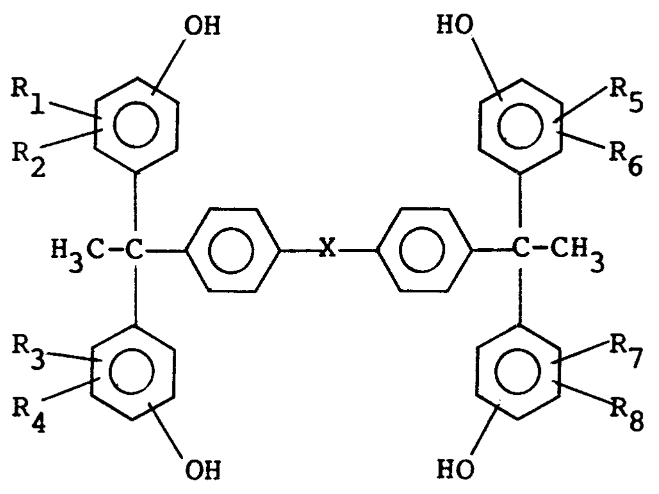

(I)

wherein $R_1$ to $R_8$ each denote a hydrogen atom or an alkyl group having 1 to 3 carbon atoms, and X denotes an oxygen atom, a sulfur atom, a methylene group or an ethylene group, with epichlorohydrin or beta-methylepichlorohydrin.

A process for producing the tetrahydric phenol compound represented by formula (I) is not particularly limited. For instance, 1 mol of a 4,4'-diacetyldiphenyl compound is reacted with 4 to 100 mols, preferably 5 to 20 mols of a monohydric phenol compound in the presence of an acid catalyst with stirring at a temperature of 20 to 130 °C, preferably 20 to 80 °C for 5 hours to 1 week. After the reaction is over, the catalyst is removed outside the system or neutralization is conducted with a base such as sodium hydroxide, potassium hydroxide, sodium carbonate, potassium carbonate or calcium hydroxide. Subsequently, water and excess monohydric phenol compound are removed under reduced pressure to provide a crude product. Dehydration and removal of phenol is carried out by an ordinary method such as washing

with a solvent, distillation, etc. When the thus obtained solid product is pulverized, recrystallized with a solvent, heated and dried for purification, a high-purity tetrahydric phenol compound can be obtained.

Incidentally, for selectively reacting the two carbonyl groups of the 4,4'-diacetyldiphenyl compound with the para-position to the hydroxyl group in the monohydric phenol compound, it is advisable to use, conjointly with the aforesaid acid catalyst, 0.05 to 0.2 mol, per mol of the 4,4'-diacetyldiphenyl compound, of an organic thiol compound such as beta-mercaptopropionic acid, 3-pyridinemethanethiol or thioethanolamine.

The monohydric phenol compound is not particularly limited. Examples thereof are phenol, o-cresol, m-cresol, xylenol and propylphenol. They may be used either singly or in combination.

The 4,4'-diacetyldiphenyl compound is not particularly limited. Examples thereof are 4,4'-diacetyldiphenylether, 4,4'-diacetyldiphenylmethane, 4,4'-diacetyldiphenylethane and 4,4'-diacetyldiphenylthioether. They may be used either singly or in combination.

Examples of the acid catalyst used in the reaction can be mineral acids such as sulfuric acid, hydrochloric acid and nitric acid, oxalic acid, p-toluenesulfonic acid, hydrogen bromide and hydrogen chloride. The amount of the acid catalyst is the same as, or larger than, the amount of 4,4'-diacetyldiphenyl compound; preferably, it is at least 8 mols per mol of the 4,4'-diacetyldiphenyl compound. An excessive amount of the acid catalyst may be used. However, as the post-treatment such as purification takes much time, the amount of the acid catalyst may properly be determined.

By the way, the dehydrocondensation reaction does not particularly require a solvent, but an inert solvent such as benzene, toluene, xylene, chloroform or tetrahydrofuran may be used.

Whether or not the tetrahydric phenol compound produced by the above process has an intended structure can be confirmed by measuring an infrared absorption spectrum, a $^{13}C$-nuclear magnetic resonance spectrum or a mass spectrum. For instance, the infrared absorption spectrum is measured to ascertain that absorption ascribable to the carbonyl group in the starting 4,4'-diacetyldiphenyl compound disappears, and the mass spectrum is measured to ascertain that the parent peak appears in the position of the intended molecular weight.

A process for producing an epoxy resin by the reaction of a tetrahydric phenol compound (I) with epichlorohydrin or beta-methylepichlorohydrin is not particularly limited. It may be carried out by e.g. a usual method wherein the addition reaction and the dehydrochlorination reaction are performed at a time using the aforesaid starting material and base.

For example, epichlorohydrin or beta-methylepichlorohydrin is added to the tetrahydric phenol compound (I), and epoxidation is conducted at 20 to 120 °C in the presence of a base. Subsequently, an aqueous layer is removed and excess epichlorohydrin or beta-methylepichlorohydrin is distilled off. An organic solvent is then added to the reaction product to dissolve it. The mixture is washed with water, and an oily layer is withdrawn. Moreover, after water is removed from the oily layer by azeotropic distillation, filtration is conducted and the solvent is distilled off to afford a final tetraepoxy resin.

When the proportion of epichlorohydrin or betamethylepichlorohydrin relative to the tetrahydric phenol compound is properly adjusted, tetraepoxy resins different in epoxy equivalent and softening point can be afforded. The amount of epichlorohydrin or beta-methylepichlorohydrin in producing the tetraepoxy resin is not particularly limited. It is usually at least 2.5 mols, preferably 5 to 15 mols per mol of the tetrahydric phenyl compound (I).

The thus obtained tetraepoxy resin is a tetraglycidyl ether compound of the tetrahydric phenol compound used as a starting material.

The tetraepoxy resin has a structure represented by formula (II).

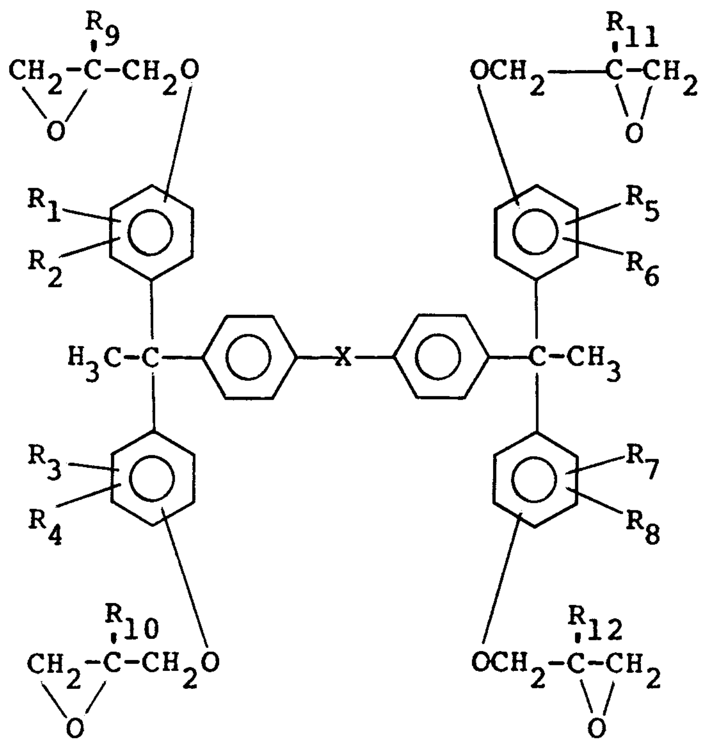

(II)

wherein $R_1$ to $R_{12}$ each denotes a hydrogen atom or an alkyl group having 1 to 3 carbon atoms, and X denotes an oxygen atom, a sulfur atom, a methylene group or an ethylene group.

The tetraepoxy resin represented by formula (II) is hereinafter simply referred to as a "tetraepoxy resin (II)".

In the process of this invention, the tetraepoxy resin (II) alone is rarely separated and usually contains trace amounts of a dimer and a trimer.

Whether or not the tetraepoxy resin obtained by the process of this invention has the structure represented by formula (II) can be confirmed by measuring an infrared absorption spectrum, $^{13}C$-nuclear magnetic resonance spectrum, a mass spectrum, an epoxy equivalent and a softening point.

For example, it is sufficient to confirm that the specific absorption ascribable to the phenolic hydroxyl group disappears in the infrared absorption spectrum, the parent peak is present in the position of the intended molecular weight in the mass spectrum, and the epoxy equivalent is approximate to the theoretical value.

A cured product can be obtained by admixing the tetraepoxy resin obtained via the process of this invention or a mixture of this and a known epoxy resin with a curing agent to form a curable composition and then heat-curing said composition.

The curing agent used to form the epoxy resin composition can be all compounds ordinarily used as the curing agent of the epoxy resin. Examples of the curing agent include aliphatic amines such as diethylenetriamine and triethylenetetramine; aromatic amines such as m-phenylenediamine, diaminodiphenylmethane and diaminodiphenyl sulfone; polyamide resins and their modified products; acid anhydrides such as maleic anhydride, phtharic anhydride, hexahydrophthalic anhydride and pyromellitic anhydride; and latent curing agents such as dicyanediamide, imidazole, $BF_3$-amine complex and guanidine derivatives.

These curing agents may be used either singly or in combination.

The amount of the curing agent is generally such that the number of the epoxy group contained in a molecule of the epoxy compound used is approximately equivalent to the number of the active hydrogen in the curing agent.

When the aforesaid compounds are employed as the curing agents, a curing accelerator can properly be used. Compounds known as curing accelerators are available. Examples thereof are tertiary amines, imidazoles, organic acid metal salts, Lewis acids and their amine complexes. They may be used either singly or in combination.

The abovementioned epoxy resin composition may further contain, if required, various additives such as

a filler, a coloring agent, a mold release agent, a fire retardant and a coupling agent. Still further, a tar, a pitch, an amino resin, phenolic, an alkyd resin, a phenolic resin, etc. can also be conjointly used.

Examples of the filler are silica, zirconium silicate, alumina, calcium carbonate, quartz powder, zirconium oxide, talc, clay, barium sulfate, asbestos powder and milled glass. Examples of the coloring agent are carbon black and other metallic compounds. Examples of the mold release agent are stearic acid, stearic acid metal salts, natural waxes and synthetic waxes. Examples of the fire retardant are antimony trioxide and hexabromobenzene. Examples of the coupling agent are acrylic silanes, amino silanes and epoxy silanes.

As the epoxy resin composition of this invention gives a cured product excellent in not only heat resistance but also toughness and water resistance, it can be used in electrical insulating materials such as semiconductor sealing materials, paint insulating varnishes, laminates and insulating powder paints; laminates for printed circuit boards; prepregs; electroconductive adhesives and adhesives for construction materials such as honeycomb panels; molding materials for electric parts except semiconductors; glass fiber-reinforced plastics, carbon fiber-reinforced plastics and their prepregs; and resist inks.

The following Examples, Comparative Examples and Referential Example illustrate this invention more specifically. Parts in said Examples are all by weight unless otherwise indicated.

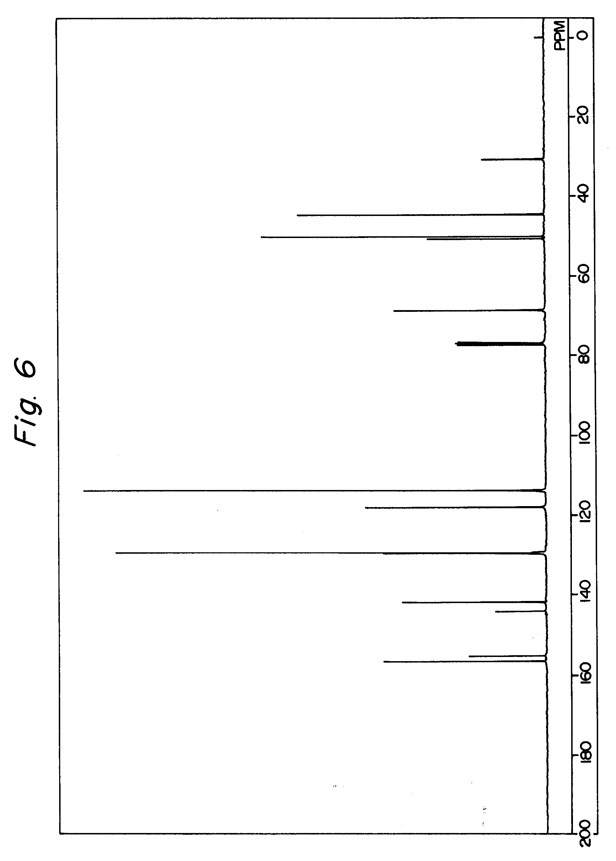

In the attached drawings, Figures 1, 2 and 3 illustrate a mass spectrum (FD-MS), an infrared absorption spectrum (IR) and a neuclear magnetic resonance spectrum (NMR) of a tetrahydric phenol compound (a) obtained in Example 1. Figures 4, 5 and 6 illustrate FD-MS, IR and NMR of an epoxy resin (a) obtained in Example 6.

EXAMPLE 1

(Synthesis of a tetrahydric phenol compound)

4,4'-Diacetyldiphenylether (495 g: 1.95 mols) was dissolved in 1,116 g (11.5 mols) of phenol, and 25 ml of beta-mercaptopropionic acid was added at room temperature. Subsequently, about 20 mols of a hydrogen chloride gas was bubbled over the course of 2 hours, and the reaction was run at 25 °C for 7 days with stirring. After the reaction was over, the reaction liquid was distilled under reduced pressure and phenol was removed. The reaction product was washed with hot water to conduct oil-water separation. There resulted a crude product. The crude product was recrystallized from an aqueous acetic acid solution (water/acetic acid weight ratio = 1/1) and dried to obtain 700 g of a product (a). A purity of the product (a) was measured by gel permeation chromatography and found to be 98 %.

A mass spectrum, an infrared absorption spectrum and a $^{13}$C-nuclear magnetic resonance spectrum were measured for the product (a). The results are shown in Figures 1 to 3 respectively. The measuring conditions of these spectra are as follows.

Mass spectrum (FD-MS)

Machine: JMX AX-505H, a double convergence mass spectrograph manufactured by JEOL, Ltd.
FD conditions: accelerating voltage - 3 KV
cathode voltage - 5 KV
emitter current - 0 to 50 mA (2 mA/min)
mass range - 20 to 2300 (1 sec, 20 sec)

Infrared absorption spectrum (IR)

Machine: IR-810, an infrared spectrophotometer manufactured by Nippon Bunko Kogyo K.K.
Condition: time - 6 sec

Nuclear magnetic resonance spectrum ($^{13}$C-NMR)

Machine: JNM.GSX-270, a high resolution nuclear magnetic resonance absorption device
Conditions: complete proton decoupling mode
pulse width - 6 sec
pulse interval - 3 sec
solvent - $CDCl_3$

The mass spectrum reveals that the product is a compound having a parent peak $M^+$ = 594 and a

fragment peak $M^{2+}$ = 297.

The infrared absorption spectrum showed the specific absorptions in the following positions.

| | |
|---|---|
| 3400 $cm^{-1}$ | -OH |
| 2950 $cm^{-1}$ | $-CH_3$ |
| 1600 $cm^{-1}$ | 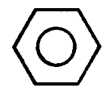 |
| 1500 $cm^{-1}$ | 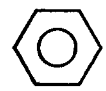 |
| 1240 $cm^{-1}$ | -O- |
| 830 $cm^{-1}$ | 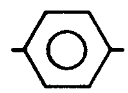 |

The specific absorption ascribable to the carbonyl group of the starting 4,4'-diacetyldiphenylether disappeared. A melting point of the product (a) was 152 to 154 °C.

From the above results, the product (a) was presumed to be 4,4'-bis[2,2,-di(hydroxyphenyl)ethyl]-phenylether.

EXAMPLE 2

(Synthesis of a tetrahydric phenol compound)

Example 1 was repeated except using 1,242 g of o-cresol instead of 1,116 g of phenol. There resulted 750 g of a product (b). A melting point of the product (b) was 121 to 123 °C. A purity of the product (b) was measured by gel permeation chromatography and found to be 95 %.

An infrared absorption spectrum was measured for the product (b).

The infrared absorption spectrum showed the specific absorptions in the following positions.

| | |
|---|---|
| 3400 $cm^{-1}$ | -OH |
| 2950 $cm^{-1}$ | $-CH_3$ |
| 1600, 1500 $cm^{-1}$ | 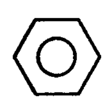 |
| 1250 $cm^{-1}$ | -O- |
| 830 $cm^{-1}$ | 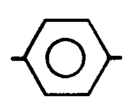 |
| 850, 800 $cm^{-1}$ | 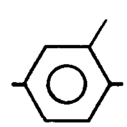 |

The specific absorption ascribable to the carbonyl group of the starting 4,4'-diacetyldiphenylether disappeared.

From the foregoing results, the product (b) was presumed to be 4,4'-bis[2,2,-di(methylhydroxyphenyl)-ethyl]phenylether.

EXAMPLE 3

(Synthesis of a tetrahydric phenol compound)

4,4'-Diacetyldiphenylmethane (491 g: 1.95 mols) was dissolved in 1,116 g (11.5 mols) of phenol, and 25 ml of beta-mercaptopropionic acid was added at room temperature. About 20 mols of a hydrogen chloride gas was then bubbled over the course of 2 hours, and the reaction was run at 25°C for 7 days with stirring. After the reaction was terminated, the reaction liquid was distilled under reduced pressure and phenol was removed. The reaction product was washed with hot water and oil-water separation was conducted. There resulted a crude product. The crude product was recrystallized from an aqueous acetic acid solution (water/acetic acid weight ratio = 1/1) and dried to afford 690 g of a product (c).

A melting point of the product (c) was 135 to 137°C. A purity of the product (c) was measured by gel permeation chromatography and found to be 98 %.

An infrared absorption spectrum was measured for the product (c).

The infrared absorption spectrum showed the specific absorptions in the following positions.

| | |
|---|---|
| 3400 $cm^{-1}$ | -OH |
| 3050, 2950, 2850 $cm^{-1}$ | $-CH_3$, $-CH_2-$ |
| 1600 $cm^{-1}$ | 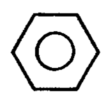 |
| 1500 $cm^{-1}$ | 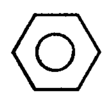 |
| 1450 $cm^{-1}$ | $-CH_2-$ |
| 830 $cm^{-1}$ | 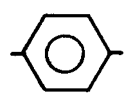 |

The specific absorption ascribable to the carbonyl group of the starting 4,4'-diacetyldiphenylmethane disappeared.

From the foregoing results, the product (c) was presumed to be 4,4'-bis[2,2,-di(hydroxyphenyl)ethyl]-phenylmethane.

EXAMPLE 4

(Synthesis of a tetrahydric phenol compound)

Example 3 was repeated except using 1,242 g of o-cresol instead of 1,116 g of phenol. There was obtained 740 g of a product (d).

A melting point of the product (d) was 131 to 133°C. A purity of the product (d) was measured by gel permeation chromatography and found to be 95 % or higher.

The infrared absorption spectrum showed the specific absorptions in the following positions.

| | |
|---|---|
| $3400\ cm^{-1}$ | $-OH$ |
| $3050 - 2850\ cm^{-1}$ | $-CH_3$, $-CH_2-$, $-\dot{C}H-$ |
| $1600, 1500\ cm^{-1}$ | 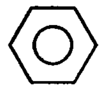 |
| $1450\ cm^{-1}$ | $-CH_2-$ |
| $830\ cm^{-1}$ | 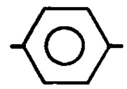 |
| $850, 800\ cm^{-1}$ | 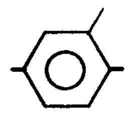 |

The specific absorption ascribable to the carbonyl group of the starting 4,4'-diacetyldiphenylmethane disappeared.

From the foregoing results, the product (d) was presumed to be 4,4'-bis[2,2,-di(methylhydroxyphenyl)-ethyl]phenylmethane.

EXAMPLE 5

(Synthesis of a tetrahydric phenol compound)

Example 3 was repeated except using 527 g of 4,4'-diacetyldiphenylthioether instead of 491 g of 4,4'-diacetyldiphenylmethane. There resulted 700 g of a product (e).

A melting point of the product (e) was 168 to 169 °C. A purity of the product (e) was measured by gel permeation chromatography and found to be 98 % or higher.

An infrared absorption spectrum was measured for the product (e).

The infrared absorption spectrum showed the specific absorptions in the following positions.

| | |
|---|---|
| $3400\ cm^{-1}$ | $-OH$ |
| $3050, 2950, 2850\ cm^{-1}$ | $-CH_3$, $-CH_2-$, $-\dot{C}H-$ |
| $1600, 1500\ cm^{-1}$ | 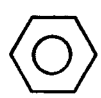 |
| $830\ cm^{-1}$ | 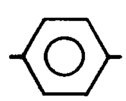 |
| $710\ cm^{-1}$ | $-S-$ |

The specific absorption ascribable to the carbonyl group of the starting 4,4'-diacetyldiphenylthioether disappeared.

From the foregoing results, the product (e) was presumed to be 4,4'-bis[2,2,-di(hydroxyphenyl)ethyl]-phenylthioether.

EXAMPLE 6

(Synthesis of an epoxy resin)

The product (a) (597 g: 1 mol) obtained in Example 1 was dissolved in 1,110 g (12 mols) of epichlorohydrin, and with stirring 880 g (4.4 mols) of a 20 wt.% sodium hydroxide aqueous solution was added dropwise thereto at 80° C over the course of 5 hours. The reaction was further performed for 1 hour. After the aqueous layer was separated, excess epichlorohydrin was distilled off. The resulting reaction product was uniformly dissolved in 985 g of methyl isobutyl ketone. Further, the mixture was washed with 410 g of water. Subsequently, oil-water separation was conducted to withdraw an oily layer. After filtration, methyl isobutyl ketone was distilled off to obtain 755 g of an epoxy resin (a).

The epoxy resin (a) was recrystallized and dried. A softening point of the epoxy resin (a) after recrystallization was 65 to 66° C. A purity of the epoxy resin (a) was measured by gel permeation chromatography and found to be 98 % or higher.

An epoxy equivalent of the epoxy resin (a) after recrystallization was 206 which was approximate to a calculated value of 204.5.

A mass spectrum, an infrared absorption spectrum, and a $^{13}C$-nuclear magnetic resonance spectrum were measured for the epoxy resin (a) after recrystallization.

The results are shown in Figures 4 to 6 respectively.

From the mass spectrum, the epoxy resin (a) is found to be a compound having a parent peak $M^+$ of 818 and a fragment peak $M^+$ of 409.

An infrared absorption spectrum of the epoxy resin (a) showed the specific absorptions in the following positions.

| | |
|---|---|
| 3050 - 2850 $cm^{-1}$ | $-CH_3$, $-CH_2-$, $-\overset{|}{C}H-$ |
| 1600, 1500 $cm^{-1}$ | 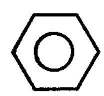 |
| 1250 $cm^{-1}$ | -O- |
| 920 $cm^{-1}$ | 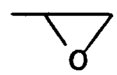 |
| 840 $cm^{-1}$ | 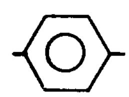 |

The specific absorption ascribable to the phenolic hydroxyl group in the starting product (a) disappeared.

From the above results, the epoxy resin (a) was presumed to be 4,4'-bis[2,2,-di(glycidyloxyphenyl)-ethyl]phenylether.

By the way, the softening point was measured as follows.

A specimen was pulverized and melted at about 150° C for about 15 minutes. A ring heated to the same temperature as the specimen was put on a smooth metallic plate and the specimen was poured thereon. Another smooth metallic plate was also put on the ring. The smooth metallic plates with the ring in between were allowed to cool to room temperature, thereby preparing a sample.

The sample was placed on a ring and ball automatic softening point measuring device (the same as a device of Walter Herjog), and a brass ball was mounted in the center of the brass ball ring. The temperature was elevated at a rate of 5° C per minute. The temperature given when the sample reached the bottom plate was made a softening point.

EXAMPLE 7

(Synthesis of an epoxy resin)

Example 1 was repeated except using 653 g of the product (b) in Example 2 instead of the product (a). There was obtained 806 g of an epoxy resin (b).

The epoxy resin (b) was recrystallized and dried. A softening point of the epoxy resin (b) after recrystallization was 70 to 73 °C. A purity of the epoxy resin (b) was measured by gel permeation chromatography and found to be 93 % or higher.

An epoxy equivalent of the epoxy resin (b) after recrystallization was 220 which was approximate to a calculated value of 219.5.

An infrared absorption spectrum was measured for the epoxy resin (b).

Said infrared absorption spectrum showed the specific absorptions in the following positions.

| | |
|---|---|
| 3050 - 2850 $cm^{-1}$ | $-CH_3$, $-CH_2-$, $-\dot{C}H-$ |
| 1600, 1500 $cm^{-1}$ | 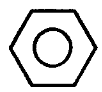 |
| 1250 $cm^{-1}$ | -O- |
| 940 $cm^{-1}$ | 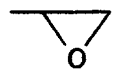 |
| 830 $cm^{-1}$ | 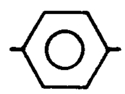 |
| 850, 800 $cm^{-1}$ | 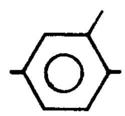 |

The specific absorption ascribable to the phenolic hydroxyl group in the starting product (b) disappeared.

From the above results, the epoxy resin (b) was presumed to be 4,4'-bis[2,2,-di-(methylglycvidyloxyphenyl)ethyl]phenylether.

EXAMPLE 8

(Synthesis of an epoxy resin)

The product (c) (595 g: 1 mol) obtained in Example 3 was dissolved in 1,110 g (12 mols) of epichlorohydrin, and 880 g (4.4 mols) of a 20 wt.% sodium hydroxide aqueous solution was added dropwise at 80 °C for 5 hours with stirring. The reaction was further conducted for 1 hour. The aqueous layer was then separated, and exces= epichlorohydrin was recovered by distillation. The resulting reaction product was uniformly dissolved in 983 g of methyl isobutyl ketone. Further, the mixture was washed with 410 g of water. Subsequently, oil-water separation was conducted to withdraw the oily layer. From the oily layer, water was removed by azeotropic distillation. After biltration, isobutyl ketone was distilled off to obtain 750 g of an epoxy resin (c).

The epoxy resin (c) was recrystallized and dried. A softening point of the epoxy resin (c) after recrystallization was 105 to 107 °C. A purity of the epoxy resin (c) was measured by gel permeation chromatography and found to be 95 % or higher.

An epoxy equivalent of the epoxy resin (c) after recrystallization was 206 which was approximate to a calculated value of 204.0.

An infrared absorption spectrum was measured for the epoxy resin (c).

Said infrared absorption spectrum showed the specific absorptions in the following positions.

| | |
|---|---|
| 3050 - 2850 $cm^{-1}$ | $-CH_3$, $-CH_2-$, $-\overset{\vert}{C}H-$ |
| 1600 $cm^{-1}$ | 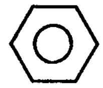 |
| 1500 $cm^{-1}$ | 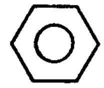 |
| 1450 $cm^{-1}$ | $-CH_2-$ |
| 920 $cm^{-1}$ | 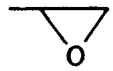 |
| 840 $cm^{-1}$ | 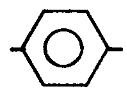 |

The specific absorption ascribable to the phenolic hydroxyl group in the starting product (c) disappeared.

From the above results, the epoxy resin (c) was presumed to be 4,4'-bis[2,2,-di(glycidyloxyphenyl)-ethyl]phenylmethane.

EXAMPLE 9

(Synthesis of an epoxy resin)

Example 8 was repeated except using 651 g of the product (c) in Example 3 instead of 595 g of the product (c) in Example 3. There was obtained 802 g of an epoxy resin (d).

The epoxy resin (d) was recrystallized and dried. a softening point of the epoxy resin after recrystallization was 107 to 109 °C. A purity of the epoxy resin (d) was measured by gel permeation chromatography and found to be 94 % or higher.

An epoxy equivalent of the epoxy resin (d) after recrystallization was 210 which was approximate to a calculated value of 207.5.

An infrared absorption spectrum was measured for the epoxy resin (d).

Said infrared absorption spectrum showed the specific absorptions in the following positions.

| | |
|---|---|
| 3050 - 2850 $cm^{-1}$ | $-CH_3$, $-CH_2-$, -CH- |
| 1600, 1500 $cm^{-1}$ | 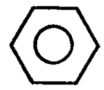 |
| 1450 $cm^{-1}$ | $-CH_2-$ |
| 830 $cm^{-1}$ | 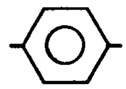 |
| 850, 800 $cm^{-1}$ | 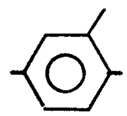 |

The specific absorption ascribable to the phenolic hydroxyl group in the starting product (c) disappeared.

From the above results, the epoxy resin (d) was presumed to be 4,4'-bis[2,2,-di(glycidyloxyphenyl)-ethyl]phenylmethane.

EXAMPLE 10

(Synthesis of an epoxy resin)

Example 9 was repeated except using 613 g of the product (d) in Example 5 instead of the product (c) in Example 4. There was obtained 770 g of the epoxy resin (e).

The epoxy resin (e) was recrystallized and dried. A softening point of the epoxy resin (e) after recrystallization was 212 to 213°C. A purity of the epoxy resin (e) was measured by gel permeation chromatography and found to be 94 % or higher.

An epoxy equivalent of the epoxy resin (e) after recrystallization was 210 which was approximate to a calculated value of 208.5.

An infrared absorption spectrum was measured for the epoxy resin (e).

Said infrared absorption spectrum showed the specific absorptions in the following positions.

| | |
|---|---|
| 3050 - 2850 $cm^{-1}$ | $-CH_3$, $-CH_2-$, -CH- |
| 1600, 1500 $cm^{-1}$ | 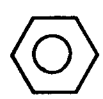 |
| 830 $cm^{-1}$ | 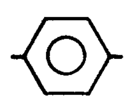 |
| 850, 800 $cm^{-1}$ | 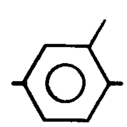 |
| 710 $cm^{-1}$ | -S- |

The specific absorption ascribable to the phenolic hydroxyl group in the starting product (d) disappeared.

From the above results, the epoxy resin (e) was presumed to be 4,4'-bis[2,2,-di(glycidyloxyphenyl)-

ethyl]phenylthioether.

EXAMPLE 11

4,4'-Bis[2,2,-di(hydroxyphenyl)ethyl]phenylether (597 g: 1 mol) obtained in Example 1 was dissolved in 740 g (8 mols), and with stirring, 880 g (4.4 mols) of a 20 wt.% sodium hydroxide aqueous solution was then added dropwise at 80 °C over the course of 5 hours. The reaction was further run for 1 hour. The aqueous layer was then separated and excess epichlorohydrin was distilled off. The resulting reaction product was uniformly dissolved in 985 g of methyl isobutyl ketone. Further, the mixture was washed with 410 g of water, and oil-water separation was then conducted to withdraw the oily layer. From the oily layer, water was removed by azeotropic distillation. Thereafter, filtration was conducted and methyl isobutyl ketone was distilled off to obtain 750 g of an epoxy resin (f) having an epoxy content of 260 and a softening point of 65 °C.

REFERENTIAL EXAMPLE

1,1,5,5-Tetrakis(hydroxyphenyl)pentane (440 g) was dissolved in 1,665 g of epichlorohydrin, and with stirring, 880 g of a 20 % sodium hydroxide aqueous solution was added dropwise at 80 °C over the course of 5 hours. While keeping the same temperature for 1 hour, the reaction continued and the aqueous layer was then separated. Excess epichlorohydrin was then recovered by distillation. The resulting reaction product was uniformly dissolved in 1,500 g of methyl isobutyl ketone (hereinafter abbreviated as "MIBK"). Thereafter, the mixture was washed with 800 g of water. Oil-water separation was conducted, and water was removed from the oily layer by azeotropic distillation. Filtration was conducted and MIBK was distilled off. There resulted 625 g of solid 1,1,5,5-tetrakis(glycidyloxyphenyl)pentane having an epoxy equivalent of 184.

EXAMPLES 12 to 17 and COMPARATIVE EXAMPLE 1

Each of the epoxy resins (a) to (f) obtained in Examples 6 to 11 and the epoxy resin [1,1,5,5-tetrakis-(glycidyloxyphenylpentane] obtained in Referential Example, EPICLON B-570 (a tradename for methyltetrahydrophthalic anhydride made by Dainippon Ink & Chemicals, Inc.) as a curing agent and benzyldimethylamine as a curing accelerator were blended as shown in Table 1 such that one acid anhydride group of the curing agent per epoxy group of the epoxy resin was present. There resulted epoxy resin compositions in Examples 12-17 and Comparative Example 1.

These epoxy resin compositions were cured by heating at 100 °C for 2 hours, at 160 °C for 2 hours and at 180 °C for 2 hours to form test pieces. A heat distortion temperature, a flexural strength, a flexural modulus, a tensile strength, a tensile elongation and a boiling water absorption were measured in accordance with JIS K-6911. The results are shown in Table 1.

EXAMPLES 18 to 23 and COMPARATIVE EXAMPLE 2

Examples 12 to 17 and Comparative Example 1 were repeated except that a novolak-type phenolic resin, BARCUM TD-2131 [a tradename for a product made by Dainippon Ink & Chemicals, Inc, sobtening point 80 °C) was used instead of EPICLON B-570 as a curing agent and the components were blended as shown in Table 1 such that one phenolic hydroxyl group of the curing agent per epoxy group of the epoxy resin was present.

These epoxy resin compositions were cured by heating at 100 °C for 2 hours, at 120 °C for 2 hours and at 150 °C for 2 hours to form test pieces. The test pieces were subjected to the same measurements as in Example 1. The results are shown in Table 1.

Table 1

| | | Example | | | | |
|---|---|---|---|---|---|---|
| | | 12 | 13 | 14 | 15 | 16 |
| Epoxy resin (a) | (parts) | 100 | — | — | — | — |
| Epoxy resin (b) | (parts) | — | 100 | — | — | — |
| Epoxy resin (c) | (parts) | — | — | 100 | — | — |
| Epoxy resin (d) | (parts) | — | — | — | 100 | — |
| Epoxy resin (e) | (parts) | — | — | — | — | 100 |
| Epoxy resin (f) | (parts) | — | — | — | — | — |
| 1,1,5,5-Tetrakis(glycidyloxyphenyl)pentane | (parts) | — | — | — | — | — |
| Curing agent: EPICLON B-570 | (parts) | 68 | 64 | 68 | 64 | 66 |
| Curing agent: BARCUM TD-2131 | (parts) | — | — | — | — | — |

- to be continued -

Table 1 (continued)

| | | Example | | | | |
|---|---|---|---|---|---|---|
| | | 12 | 13 | 14 | 15 | 16 |
| Benzyldimethylamine | (parts) | 0.5 | 0.5 | 0.5 | 0.5 | 0.5 |
| Heat distortion temperature | (°C) | 194 | 196 | 192 | 194 | 195 |
| Flexural strength | ($kg/mm^2$) | 13.5 | 13.6 | 13.3 | 13.5 | 13.2 |
| Flexural modulus | ($kg/mm^2$) | 390 | 410 | 380 | 400 | 410 |
| Tensile strength | ($kg/mm^2$) | 7.9 | 7.8 | 7.5 | 7.4 | 7.6 |
| Tensile elongation | ( % ) | 4.5 | 4.2 | 4.4 | 4.2 | 4.6 |
| Boiling water absorption (%) | 1-hour boiling | 0.06 | 0.05 | 0.06 | 0.05 | 0.06 |
| | 2-hour boiling | 0.17 | 0.11 | 0.17 | 0.15 | 0.17 |
| | 5-hour boiling | 0.19 | 0.17 | 0.19 | 0.18 | 0.21 |

- to be continued -

Table 1 (continued)

| | | Example | Comparative Example | Example | |
|---|---|---|---|---|---|
| | | 17 | 1 | 18 | 19 |
| Epoxy resin (a) (parts) | | — | — | 100 | — |
| Epoxy resin (b) (parts) | | — | — | — | 100 |
| Epoxy resin (c) (parts) | | — | — | — | — |
| Epoxy resin (d) (parts) | | — | — | — | — |
| Epoxy resin (e) (parts) | | — | — | — | — |
| Epoxy resin (f) (parts) | | 100 | — | — | — |
| 1,1,5,5-Tetrakis(glycidyloxyphenyl)pentane (parts) | | — | 100 | — | — |
| Curing agent | EPICLON B-570 (parts) | 64 | 90 | — | — |
| | VARCUM TD-2131 (parts) | — | — | 42 | 40 |

- to be continued -

Table 1 (continued)

| | | Example | Comparative Example | Example | |
|---|---|---|---|---|---|
| | | 17 | 1 | 18 | 19 |
| Benzyldimethylamine (parts) | | 0.5 | 0.5 | 0.5 | 0.5 |
| Heat distortion temperature (°C) | | 186 | 178 | 199 | 201 |
| Flexural strength ($kg/mm^2$) | | 13.2 | 12.9 | 14.1 | 14.1 |
| Flexural modulus ($kg/mm^2$) | | 390 | 375 | 520 | 540 |
| Tensile strength ($kg/mm^2$) | | 7.6 | 7.5 | 9.7 | 8.9 |
| Tensile elongation ( % ) | | 5.0 | 4.2 | 4.8 | 4.4 |
| Boiling water absorption (%) | 1-hour boiling | 0.06 | 0.09 | 0.05 | 0.04 |
| | 2-hour boiling | 0.17 | 0.22 | 0.16 | 0.14 |
| | 5-hour boiling | 0.19 | 0.32 | 0.18 | 0.17 |

- to be continued -

Table 1 (Continued)

| | | Example | | |
|---|---|---|---|---|
| | | 20 | 21 | 22 |
| Epoxy resin (a) | (parts) | — | — | — |
| Epoxy resin (b) | (parts) | — | — | — |
| Epoxy resin (c) | (parts) | 100 | — | — |
| Epoxy resin (d) | (parts) | — | 100 | — |
| Epoxy resin (e) | (parts) | — | — | 100 |
| Epoxy resin (f) | (parts) | — | — | — |
| 1,1,5,5-Tetrakis(glycidyloxyphenyl)pentane | (parts) | — | — | — |
| Curing agent EPICLON B-570 | (parts) | — | — | — |
| Curing agent VARCOM TD-2131 | (parts) | 42 | 40 | 41 |

- to be continued -

Table 1 (Continued)

| | | Example | | |
|---|---|---|---|---|
| | | 20 | 21 | 22 |
| Benzyldimethylamine (parts) | | 0.5 | 0.5 | 0.5 |
| Heat distortion temperature (°C) | | 197 | 199 | 200 |
| Flexural strength ($kg/mm^2$) | | 13.9 | 13.9 | 13.8 |
| Flexural modulus ($kg/mm^2$) | | 510 | 520 | 550 |
| Tensile strength ($kg/mm^2$) | | 9.5 | 8.7 | 9.5 |
| Tensile elongation ( % ) | | 4.6 | 4.4 | 4.9 |
| Boiling water absorption (%) | 1-hour boiling | 0.05 | 0.04 | 0.05 |
| | 2-hour boiling | 0.16 | 0.13 | 0.16 |
| | 5-hour boiling | 0.18 | 0.16 | 0.19 |

- to be continued -

Table 1 (Continued)

| | | Example | Comparative Example |
|---|---|---|---|
| | | 23 | 2 |
| Epoxy resin (a) | (parts) | — | — |
| Epoxy resin (b) | (parts) | — | — |
| Epoxy resin (c) | (parts) | — | — |
| Epoxy resin (d) | (parts) | — | — |
| Epoxy resin (e) | (parts) | — | — |
| Epoxy resin (f) | (parts) | 100 | — |
| 1,1,5,5-Tetrakis(glycidyloxyphenyl)pentane | (parts) | — | 100 |
| Curing agent: EPICLON B-570 | (parts) | — | — |
| Curing agent: VARCOM TD-2131 | (parts) | 40 | 56 |

- to be continued -

Table 1 (Continued)

| | | Example | Comparative Example |
|---|---|---|---|
| | | 23 | 2 |
| Benzyldimethylamine (parts) | | 0.5 | 0.5 |
| Heat distortion temperature (°C) | | 190 | 181 |
| Flexural strength ($kg/mm^2$) | | 13.9 | 13.1 |
| Flexural modulus ($kg/mm^2$) | | 520 | 380 |
| Tensile strength ($kg/mm^2$) | | 9.3 | 7.7 |
| Tensile elongation ( % ) | | 5.3 | 4.4 |
| Boiling water absorption (%) | 1-hour boiling | 0.05 | 0.09 |
| | 2-hour boiling | 0.16 | 0.21 |
| | 5-hour boiling | 0.18 | 0.27 |

As shown in said Examples, the conventional epoxy resins only give the cured products which meet two of the heat resistance, the toughness and the water resistance, whereas the epoxy resins of ths invention bring forth the outstanding effects that they give the cured products which meet all of the three properties.

**Claims**

**1.** An epoxy resin represented by the formula

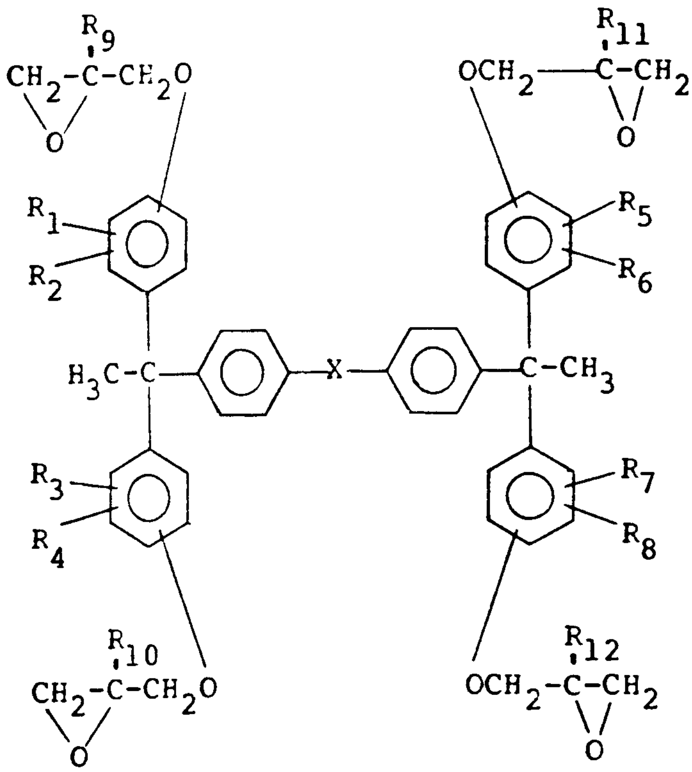

wherein $R_1$ to $R_{12}$, which may be the same or different, each denote a hydrogen atom or an alkyl group having 1 to 3 carbon atoms, and X denotes an oxygen atom, a sulfur atom, a methylene group or an ethylene group.

2. An epoxy resin according to claim 1 wherein X is an oxygen atom, $R_1$, $R_3$, $R_5$ and $R_7$ are the same and are all hydrogen atoms or methyl groups, and $R_2$, $R_4$, $R_6$, $R_8$, $R_9$, $R_{10}$, $R_{11}$ and $R_{12}$ are the same and are all hydrogen atoms.

3. A process for producing a polyepoxy resin by the reaction of a tetrahydric phenol compound with epichlorohydrin or beta-methylepichlorohydrin, characterized in that the tetrahydric phenol compound is a tetrahydric phenol compound represented by the formula

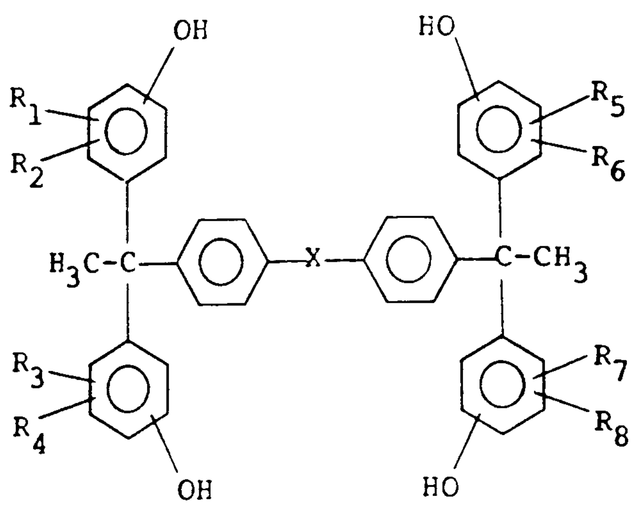

wherein $R_1$ and $R_8$ and X are as defined in claim 1 or 2.

4. An epoxy resin composition comprising an epoxy resin and a curing agent, characterized in that the epoxy resin is a polyepoxy resin as defined in claim 1 or 2.

5. A tetrahydric phenol compound as defined in claim 1 or 2.

6. A process for producing a 4,4-di[2,2,2',2'-bis(hydroxyaryl)ethyl]phenyl compound, which comprises reacting a 4,4'-diacetyldiphenyl compound with a monohydric phenol compound.

7. A process according to claim 6 wherein the 4,4'-diacetyldiphenyl compound is at least one of 4,4'-diacetyldiphenylether, 4,4'-diacetyldiphenylthioether and 4,4'-diacetyldiphenylmethane.

8. A process according to claim 6 or 7 wherein the monohydric phenol compound is phenol or cresol.

Fig. 1

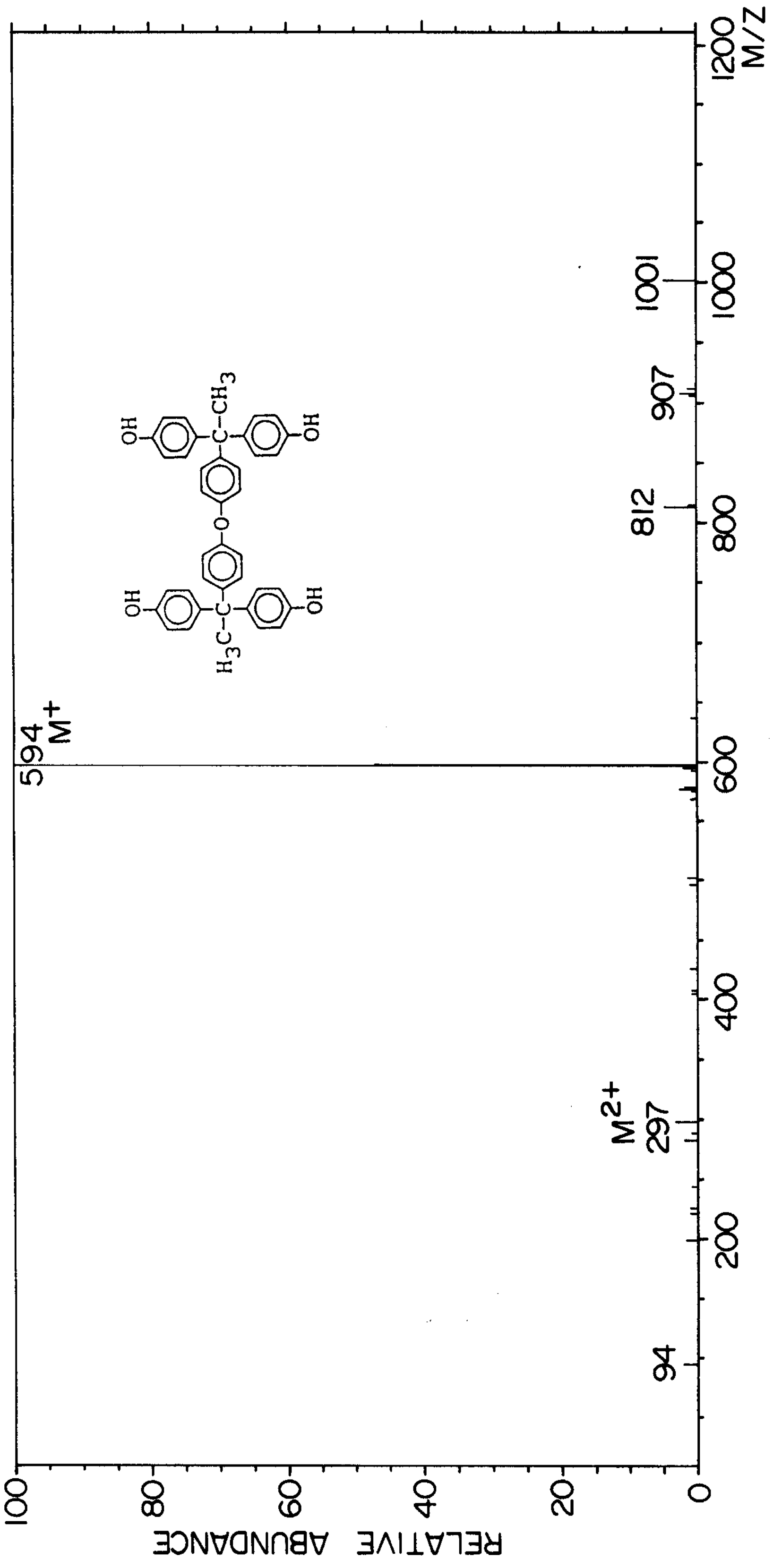
RELATIVE ABUNDANCE
100
80
60
40
20
0
M/Z
200
400
600
800
1000
1200
94
297
M2+
594
M+
812
907
1001

Fig. 2

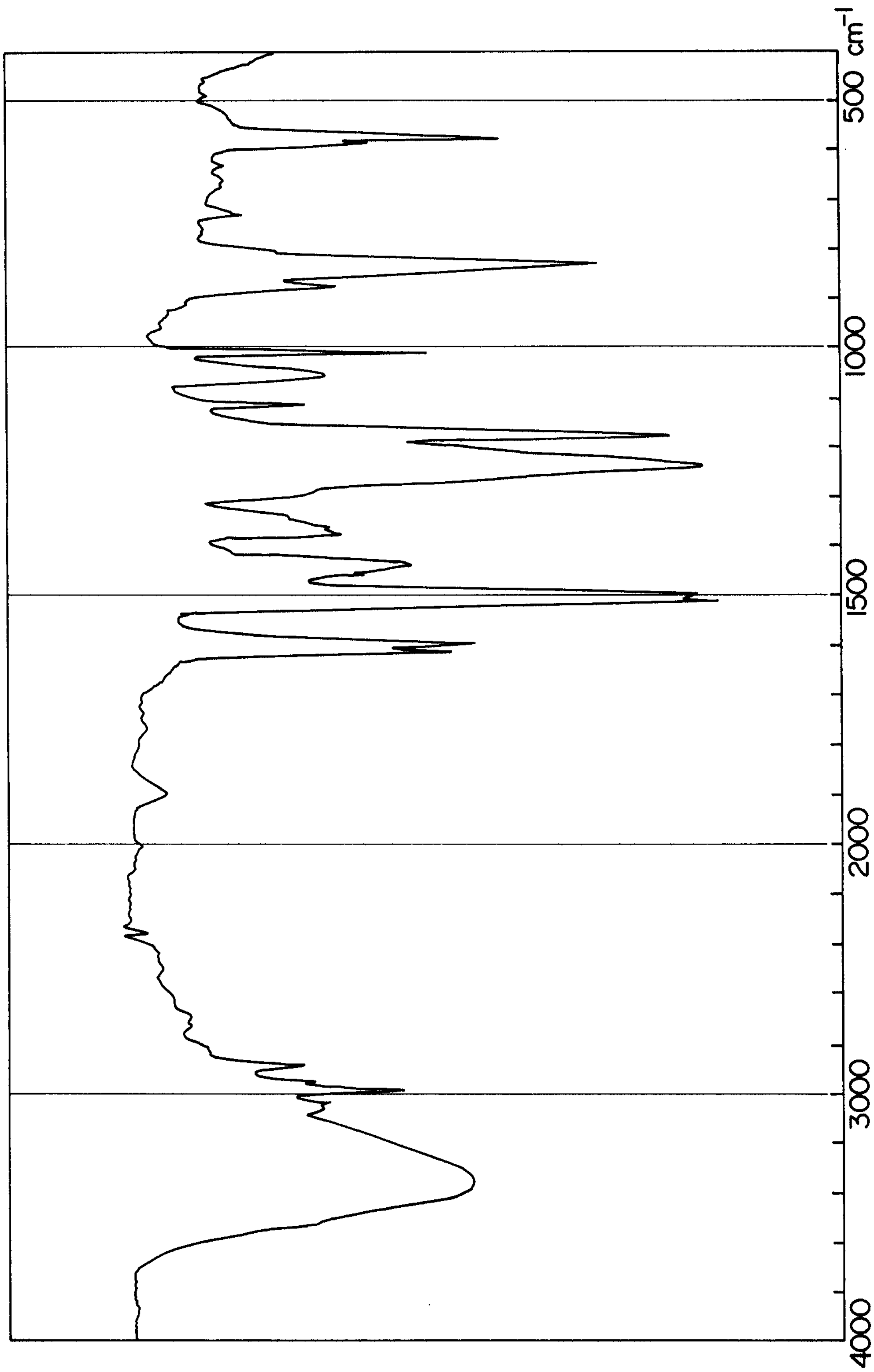

Fig. 3

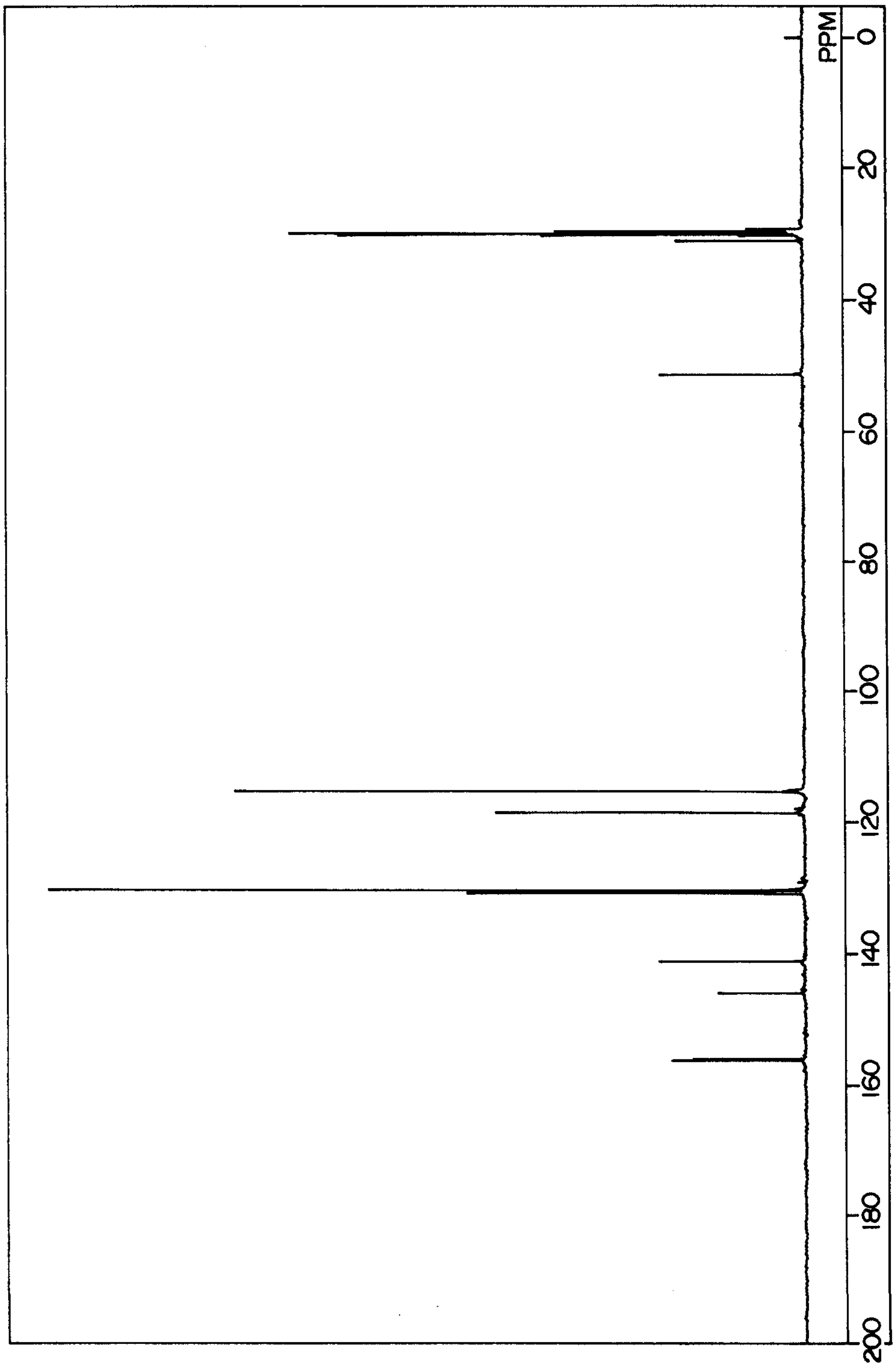

Fig. 4

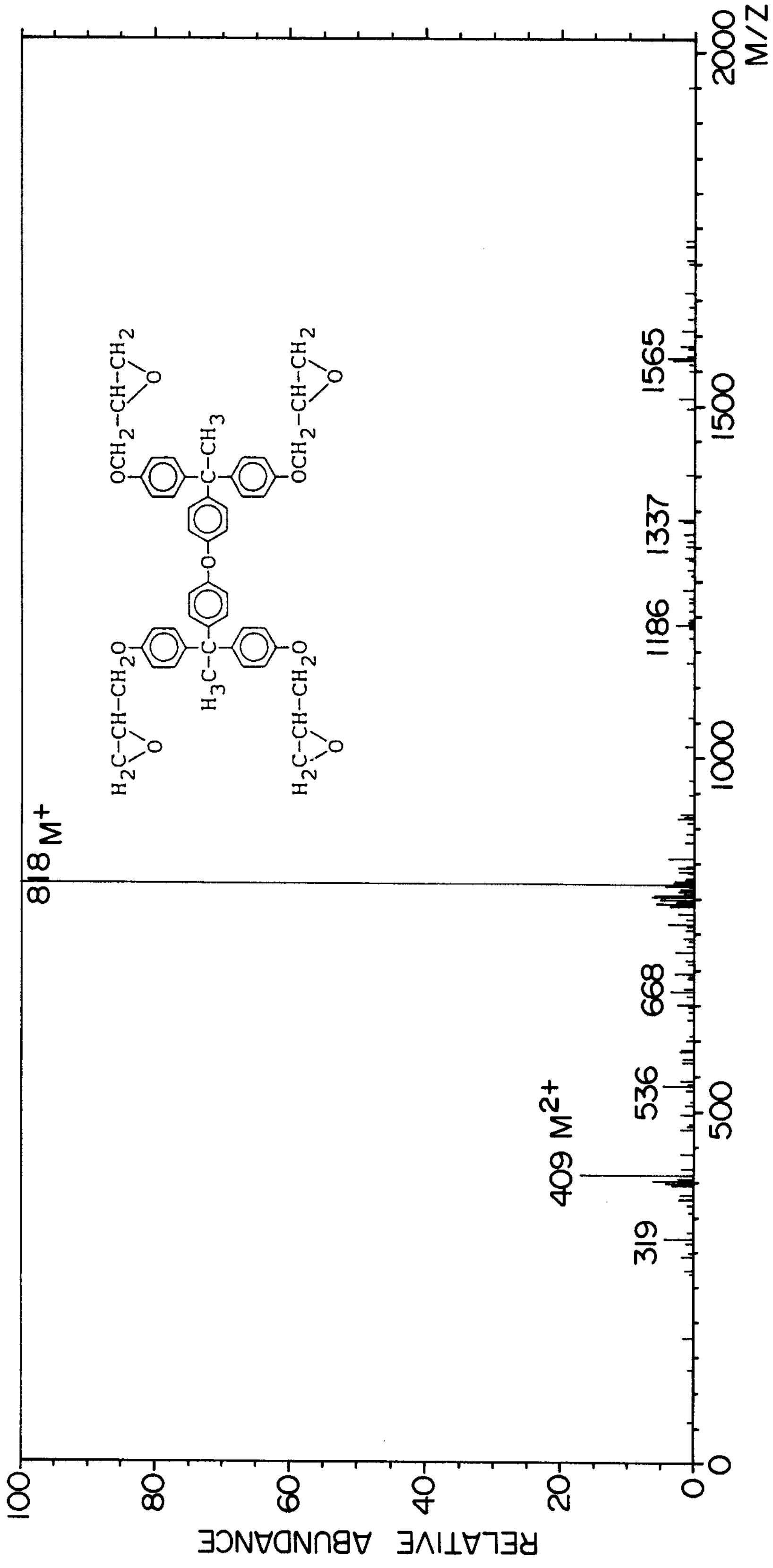
RELATIVE ABUNDANCE
M/Z
818 M+
409 M2+
319
536
668
1186
1337
1565
0
20
40
60
80
100
500
1000
1500
2000

Fig. 5

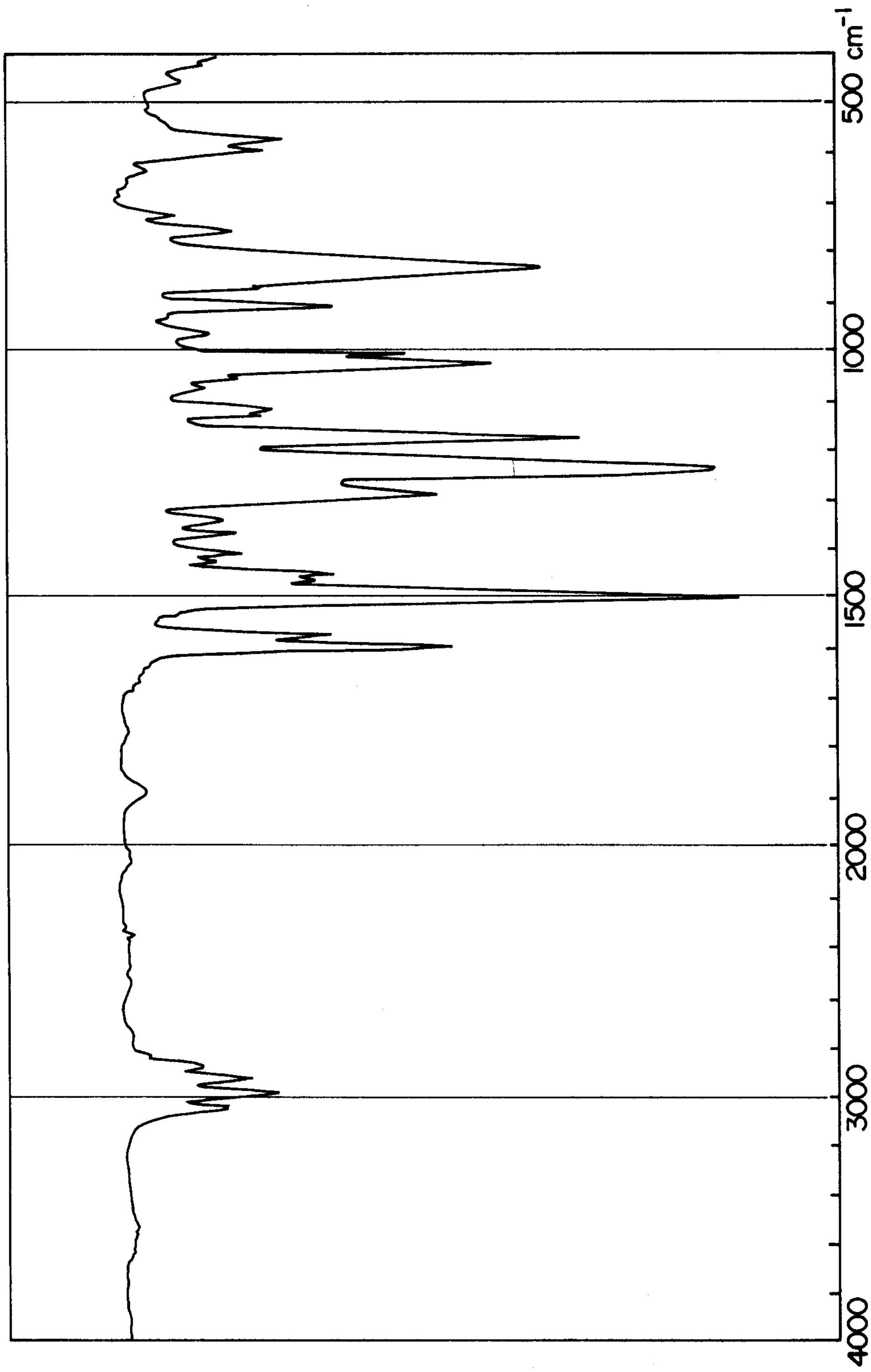

Fig. 6